(19) Europäisches Patentamt
European Patent Office
Office européen des brevets

(11) **EP 1 453 563 B1**

(12) **EUROPEAN PATENT SPECIFICATION**

(45) Date of publication and mention of the grant of the patent:
**25.07.2007 Bulletin 2007/30**

(21) Application number: **01985357.1**

(22) Date of filing: **26.11.2001**

(51) Int Cl.:
*A61M 15/00* *(2006.01)*          *B65D 83/14* *(2006.01)*

(86) International application number:
**PCT/EP2001/014184**

(87) International publication number:
**WO 2003/045482 (05.06.2003 Gazette 2003/23)**

(54) **INHALATION DEVICE**

INHALATIONSGERÄT

INHALATEUR

(84) Designated Contracting States:
**AT BE CH CY DE DK ES FI FR GB GR IE IT LI LU MC NL PT SE TR**

(43) Date of publication of application:
**08.09.2004 Bulletin 2004/37**

(73) Proprietor: **Bodrapan I/S**
**2630 Taastrup (DK)**

(72) Inventors:
  • **DRACHMANN, Bo**
  **DK-1416 Kobenhavn K (DK)**
  • **ANDERSEN, Per**
  **DK-2630 Taastrup (DK)**

(74) Representative: **Jörgensson, Leif Sixten**
**Awapatent AB**
**P.O. Box 5117**
**200 71 Malmö (SE)**

(56) References cited:
  **EP-A- 0 938 908**          **GB-A- 2 293 110**
  **US-A- 4 534 343**          **US-A- 5 042 467**
  **US-A- 5 816 240**          **US-A- 6 039 042**
  **US-B1- 6 293 279**

**Description**

Technical Field

The present invention concerns a gas inhalation device. The invention in particular concerns a gas inhalation device, which is breath-activated, for use with a gas canister according to the preamble of claim 1.

Technical Background

A basic purpose of many inhalation devices is to provide means for ensuring the administration of a drug in a way establishing physical contact between the drug and the airways, and preferably the lower airways or fine receptors of the lungs, of a patient to be treated. Note that while the present invention was developed with asthma inhalation devices in mind, the present devices may naturally be used for administration of drugs for other diseases.

Two technologies are in widespread use in asthma inhalation devices. One is powder' based, the other is gas canister based. Powder devices suffer from the drawback that the particle sizes used are relatively large, making it difficult for them to reach the fine receptors of the lungs. Gas devices provide finer particles, but eject these at a relatively high speed. A typical gas device will by activation in air create a small cloud having a length of typically 30-40 cm. On use such a cloud is likely to get in physical contact with the inner part of the mouth, i.e. the palate, rather than the fine receptors of the lungs where it is intended. A short description of the airways may elucidate this point. The upper airways include the oropharynx and larynx. The lower airways start in the trachea, which is followed by successive bifurcation into bronchi and bronchioli. Down to this point, the spaces are called conductive airways. The terminal bronchioli then divide into respiratory bronchioli until the ultimate respiratory zone, the alveoli, is reached. There is a very steep increase in the surface area with successive airways generations with consequences for drug absorption.

The inventors of the present invention have realised, that a problem occuring with the use of the known gas inhalation devices is achieving an appropriate speed of drug on inhalation. If the speed is too high, a high proportion of the drug is shot directly into the roof of the mouth, i.e. the palate. If the speed is too slow only a small portion of the drug will make it to the fine receptors of the lungs. It is usually regarded desirable to provide a large percentage of the drug in physical contact with the fine receptors of the lunges. i.e. increasing the contact area between drug and alveoli.

As an example of a powder inhaler WO 00/64520 describes a device for two or more medicaments. The powder is administered to the patient when the patient sucks from the mouthpiece and inhales the powder.

As an example of a gas canister based device EP 0 311 770 B1 describes a respiratory therapy device with a large aerosol vortexing chamber. This patent describes the beneficial effects of using a slow aerosol flow. Administration is performed when the patient inhales from a mouthpiece through a valve from the large aerosol vortexing chamber.

Another problem associated with known inhalation devices is that many of them demand coordination of breathing rhythm, activation of the device and application of the device to the mouth of the patient. Such coordination may be difficult, especially for a patient struggling to get air, and for children and aged people. It is desirable to facilitate such coordination in order to optimise administration of the drugs.

Other examples of gas inhalation devices are known from US 6 293 279, US 6 039 042, EP 0 938 908, US 5 042 467 and US 4 534 343. These documents all describe inhalation devices usable for medical purposes.

According to an aspect of the present invention it is an object of the present invention to provide means for enhancing the probability of a therapeutically effective amount of active substance reaches the fine receptors of the lungs.

According to another aspect of the present invention it is an object of the present invention to provide means for allowing a patient and/or an attending physician to achieve better control of the inhalation process and/or the way a drug is administered.

Some desirable aspects of asthma devices provided according to the present invention may be summarized as: high percentage of drug administered to patient; adequate, adjustable speed of gas on inhalation; easy adjustment of pressure without need of changing gas canister (e.g. by multiple activation of gas canister); utility with many existing gas canisters on the market; portability of device, due to size and design; discrete design; easy use; low price of device; and/or secure locking mechanism to prevent unwanted release of gas.

Disclosure of the Invention

The present invention concerns a device for the inhalation of at least one drug, from a gas canister having a nozzle, by a person suffering from asthma or bronchospasm, said device comprising: a gas container having at least one wall, said wall surrounding a gas containment chamber adapted to contain gas at a pressure larger than atmospheric pressure, said gas container further having an inlet adapted for connection to the nozzle of the gas canister, said gas container further having an outlet with an outlet valve adapted to allow the person to inhale gas from said device through said outlet; wherein said outlet valve is adapted to be activated by the inhalation of the person independent of the activation of the gas canister, and wherein the device comprises sliding means connected to said outlet valve and sliding means being adapted to slide with respect to said wall of said gas container upon activation of said outlet valve.

This aspect allows the person to activate the gas canister, and pause before inhalation. Gas from the gas canister will be contained in the gas containment chamber at a pressure larger than atmospheric pressure. On inhalation the outlet valve may be activated. The gas contained in the gas containment chamber will expand rapidly and enter the airways of the person on activation of the outlet valve. Thus, this aspect may facilitate coordination for the patient inhaling. This encourages the patient to perform a slow, deep inhalation, for maximum, deep-lung delivery of the active substance (drug). The sliding means may help keeping the outlet valve functioning correctly, as it ensures correct location of the different parts of the outlet valve upon opening and closing.

Existing, commercially available gas canisters, which may be used with the present invention, are usually provided with a nozzle. Activation of the gas canister is done by pressing or forcing the nozzle in direction towards the gas canister, whereby a dosis of active drug is emitted from the nozzle. A usual gas canister contains propellant for a higher number of doses than the number of doses of active drug in the gas canister. A vent inside the gas canister ensures that an approximately constant volume of active drug is administered on each activation of the gas canister, until the active drug has been used. The gas canisters may be any suitable gas ganisters, and inter alia contain drugs such as Bricanyl, Salbutamol, and Beclometasom.

The device according to the invention may be made from any suitable material, inter alia from metal or plastic by e.g. moulding, casting or lathing. Especially preferred materials are discussed below.

The invention may further concerns a device comprising a mouthpiece adapted to allow the person to inhale from said mouthpiece, said mouthpiece being in flow communication with said outlet valve. A mouthpiece facilitates the inhalation through the mouth of the patient.

The invention may further concerns a device provided with a removable spacer for said mouthpiece, said spacer allowing prolonging said mouthpiece on mounting. Some patients prefer to have such a spacer inserted on use, and such a spacer may be recommended for children and aged people.

The invention may further concerns a device comprising an exhalation valve; wherein said mouthpiece is in flow communication with said exhalation valve, allowing the person to exhale through the mouthpiece. This aspect further facilitates coordination of activation of gas, exhalation and subsequent inhalation of active substance, as it is not necessary to remove the mouth from the mouthpiece when exhaling.

The invention may further concerns a device, wherein said inlet is provided with receiving means being connectable to said inlet of said gas container, wherein said receiving means is adapted to receive the nozzle of the gas canister. This aspect may allow easy adaptation to different gas canisters, simply by changing the receiving means according to the selected gas canister.

The invention may further concerns a device having gas canister holding means connected to said gas container, said gas canister holding means being adapted to receiving and holding the gas canister. This aspect ensures stable, secure mounting of the gas canister.

The invention may further concerns a device; wherein the atmosphere surrounding said device has a pressure, $P_{atm}$; said outlet valve experiences an inner pressure from said gas containment chamber before activation of said outlet valve, $P_{ca}$; and said outlet valve experiences an outer pressure before activation of said outlet valve, $P_{ma}$; wherein said outlet valve is adapted to have an outlet valve parameter, *OVP,* larger than 1; said outlet valve parameter, *OVP,* being defined by the condition for activation, *B,* of said outlet valve:

$$B: \quad |P_{ca} - P_{atm}| - OVP\,|P_{atm} - P_{ma}| < 0 \text{ atm};$$

such that said outlet valve of said container is activated when said condition for activation, *B*, is fulfilled.

This aspect allows a small under pressure, with respect to atmospheric pressure, created by the patient, to be sufficient to activate the outlet valve, while a larger overpressure, with respect to atmospheric pressure, in the gas containment chamber, does not activate the outlet valve. For the parameter *OVP* being larger than 1 the outlet valve is activated by a smaller underpressure in e.g. the mouthpiece, rather than by a larger overpressure in the gas containment chamber. The parameter *OVP* may be adjusted e.g. by altering the design of the outlet valve.

The parameter *OVP* for a given device according to the invention may be measured by filling the gas containment chamber with gas at a given pressure, $P_{ca}$, e.g. with a gas canister. Subsequently, the pressure, $P_{ma}$, exerting a force on the outside of the outlet valve is lowered slowly, while measuring this pressure, $P_{ma}$. When the valve is activated by the pressure difference across the outlet valve, the parameter *OVP* may to a good approximation be calculated as:

$$OVP \approx | P_{ca} - P_{atm} | / | P_{atm} - P_{ma} |.$$

The slower the pressure $P_{ma}$ is lowered, the more accurate measurement of *OVP* may be achieved.

In order to elucidate the significance of the *OVP* parameter we offer an example below. Assume a device according to the invention has an *OVP* = 2. The pressure surrounding the device is about 1 atm. Further assume that activation of the gas canister raises the pressure to 1.3 atm inside the gas containment chamber. Before the patient inhales from the device the outer pressure, which the outlet valve experiences, is about 1 atm. Thus, we see that the condition for activation

$$B: \qquad | P_{ca} - P_{atm} | - OVP | P_{atm} - P_{ma} | < 0 \text{ atm}$$

is not fulfilled, by inserting:

$$| 1.3 - 1 | - 2 * | 1 - 1 | = 0.3 \text{ (atm)}.$$

Now, when the patient begins to inhale, the $P_{ma}$ is lowered. We see that $P_{ma} < 0.85$ is necessary to activate the outlet valve, as for $P_{ma}$ = 0.85 atm:

$$| 1.3 - 1 | - 2 * | 1 - 0.85 | = 0.0 \text{ (atm)}.$$

Evidently, higher values of the *OVP* parameter are desirable, if it is wanted that the outlet valve should be easier to activate for the patient. The higher the value, the less pressure has to be lowered on inhalation, in order to activate the outlet valve. As another example assume *OVP* = 500, while the rest of the conditions listed above remain unchanged. We see that:

$$| 1.3 - 1 | - 500 * | 1 - 0.9994 | = 0.0 \text{ (atm)},$$

i.e. $P_{ma} < 0.9994$ is sufficient for activating the outlet valve.

One way to achieve an *OVP* parameter larger than 1 is to allow the outer pressure to exert a force on a larger area of the outlet valve, than the area of the outlet valve, which the gas in the gas containment chamber is allowed to exert a force on. Thus, the outlet valve may have a large area, with respect to the patient, and a small area, with respect to the gas containment chamber.

The invention may further concerns a device, wherein said outlet valve is adapted to have an outlet valve parameter, *OVP,* larger than a number selected among the group consisting of 20; 15; 10; 8; 6; 5; 4; 3; 2.5; 2; 1.5; 1.4; 1.3; 1.2 and 1.1. The appropriate choice of the parameter *OVP* may be obtained easily by routine experiment and adapted to the preferences of the patient.

The invention may further concerns a device, wherein said outlet valve comprises a valve pin, spring means and spring attachment means; said spring means being connected to said gas container, and via said spring attachment means being connected to and forcing said valve pin to close said outlet valve except on inhalation, and allowing said outlet valve to open upon inhalation.

A pressure pin allows a small underpressure in the mouthpiece to activate the outlet valve, while the outlet valve is not activated by a larger than atmospheric pressure in the gas containment chamber.

The invention may further concerns a device, wherein said spring means are rubber bands. Alternatively a metallic, plastic or rubber spring may be used.

The invention may further concerns a device, wherein the gas in said gas containment chamber exerts a force on said outlet valve on a first area, $A_1$, and the outer pressure exerts a force on the outlet valve on a second area, $A_2$, said first area being smaller than said second area; $A_1 < A_2$. This aspect may provide a parameter, *OVP,* larger than 1.

The invention may further concerns a device, wherein the ratio, $A_2/A_1$, between said second area, $A_2$, and said first area, $A_1$, is larger than a number selected among the group consisting of 5000; 2000; 1500; 1000; 500; 300; 100;

50; 30; 20; 10; 5; 4; 3; 2 and 1.5. The larger the ratio, the easier it will be to inhale gas from the device. Thus, this aspect facilitates inhalation.

The invention may further concerns a device, said device comprising an outer wall and spacing means for connecting said gas container with said outer wall; wherein said outlet valve comprises an exhalation valve having a resilient flap valve with a circumference in close proximity to said outer wall, said resilient flap valve being adapted to allow air to be exhaled without activation of said outlet valve, and allowing activation of said outlet valve upon inhalation; and said resilient flap valve having an inhalation opening through which gas from said gas containment chamber may pass, allowing gas to flow from the gas containment chamber to the patient upon inhalation.

This aspect provides a simple valve allowing both inhalation and exhalation with the device. The resilient flap valve may be made of e.g. rubber or plastic. Especially preferred is the material silicone.

The invention may further concerns a device, wherein the force necessary to bend said resilient flap valve in direction towards the person is larger than the force necessary to bend said resilient flap valve in direction away from the person. This aspect provides easy exhalation through the resilient flap valve, and easy activation of the outlet valve upon inhalation.

The invention may further concerns a device, wherein at least part of the resilient flap is convex with respect to the person. This aspect may provide easy exhalation through the resilient flap valve, and easy activation of the outlet valve upon inhalation.

The invention may further concerns a device, wherein at least part of the resilient flap is concave with respect to the direction of the gas canister- This aspect may further provide easy exhalation through the resilient flap valve, and easy activation of the outlet valve upon inhalation.

The invention may further concerns a device, wherein said resilient flap valve allows air to flow substantially freely on exhalation, while only allowing a limited stream of air to pass between said circumference of said resilient flap valve and the inner surface of said outer wall upon inhalation. The rigidity and dimensions of the resilient flap is easily adjusted by the person skilled in the art to achieve this. This may provide for a gas cloud which is limited and does not broaden too much.

The invention may further concerns a device, wherein said sliding means surrounds said outer surface of said wall of said gas container completely, in a substantially air tight way. This aspect may hinder gas from escaping out in a direction different from the direction of the airways of the person.

The invention may further concerns a device, said device comprising an outer wall shaped as a tube with a diameter substantially equal to the diameter of the gas canister, said outer wall surrounding said inlet and said outlet of said gas container, and said device comprising spacing means for connecting said gas container with said outer wall; wherein said outer wall is connected to said mouthpiece.

A circular cross section of the device avoids sharp corners. Other suitable shapes are substantially oval, rectangular, or quadratic cross section, for allowing placement of the device on a surface without unintended rolling.

The invention may further concerns a device, wherein said outer wall further surrounds at least part of the gas canister. This aspect conceals and supports the gas canister.

The invention may further concerns a device, wherein said outer wall is provided with at least one opening situated between said inlet and said outlet of said gas container, for allowing air to be let out of the device upon exhalation. According to this aspect the patient may exhale through the mouthpiece.

The invention may further concerns a device, said device comprising an outer cover, said outer cover covering at least part of the bottom part of the gas canister, wherein said cover is adapted as to closely fit the gas canister and allow movement of said outer cover with respect to said gas container, such that the gas container may be activated by movement of said cover. This aspect will cover at least part of the gas canister, thereby providing a more discreet look of the device.

The invention may further concerns a device, wherein said outer cover is provided with a clip allowing fixation of the device to clothes. Suitable materials for such a clip are resilient materials, e.g. metal or plastic.

The invention may further concerns a device, wherein the gas canister is activated by rotation of said outer cover with respect to said gas container. This aspect provides easy activation of the gas canister, while minimizing the risk of accidental activation when not intended.

The invention may further concerns a device, wherein the outer cover comprises an elongated opening, said elongated opening having at least a part slanted with respect to the longitudinal direction of the gas canister, and said device further comprises an activation pin connected to said gas container, said pin engaging said elongated opening so that rotation of said outer cover with respect to said gas container activates said gas canister.

This aspect may be achieved by allowing a pin connected to the gas canister to stick through an at least partly helix shaped opening in a cover for the gas ganister. Rotation of the cover may then force the gas canister to be moved towards the mouthpiece.

The invention may further concerns a device, wherein the outer surface of said device is shaped as a cylinder. This aspect may provide an inhalation device of convenient shape and size, which may look like an ordinary thick pen.

Thus, a user may carry the device without attracting unwanted attention, as what might appear to be a pen is really an "Asthmapen".

The invention may further concerns a device, wherein the gas containment chamber is adapted to contain gas at a pressure larger than a pressure selected among the group consisting of 1.05; 1.1; 1.2; 1.3; 1.5; 1,6; 1.7; 1.8; 1.9; 2; 2.5 and 3 atm.

Appropriate choice of the selected pressure will inter alia depend on the size of the gas containment chamber, the pressure of the gas canister, and the choice of valves. An appropriate choice of this pressure will help ensure a large fraction of drug reaches the fine receptors of the lungs. The pressure may be selected according to the needs of the individual patient.

The invention may further concerns a device, wherein the gas containment chamber is adapted to contain gas at a pressure smaller than a pressure selected among the group consisting of 4; 3; 2.5; 2; 1.5; 1.4; 1.3; 1.2 and 1.1 atm. It may be desirable to avoid very large pressures, in order not to get a large fraction of drug adhered to the palate.

The invention may further concerns a device, wherein the gas containment chamber has dimensions which reduces the speed of gas leaving the chamber to a fraction of the speed of gas entering the chamber.

According to an aspect of the present invention the present device is used for slowing the speed of gas from the gas canister before entering the mouth of the patient, in order not to get a large fraction of drug adhered to the palate.

The invention may further concerns a device, wherein the gas containment chamber has dimensions which reduces the speed of gas leaving the chamber to a fraction of the speed of gas entering the chamber, said fraction being smaller than a number selected among the group consisting of 0.9; 0.8; 0.7; 0.6 and 0.55. Appropriate selection of this fraction may ensure high probability of a therapeutically effective amount of drug reaching the fine receptors of the lungs.

The invention may further concerns a device, wherein the gas containment chamber has dimensions which reduces the speed of gas leaving the chamber to a fraction of the speed of gas entering the chamber, said fraction being larger than a number selected among the group consisting of 0.1; 0.2; 0.3; 0.4 and 0.45. Appropriate selection of this fraction may ensure high probability of a therapeutically effective amount of drug reaching the fine receptors of the lungs.

The invention may further concerns a device, wherein the gas containment chamber has dimensions which reduces the speed of gas leaving the chamber to a fraction of the speed of gas entering the chamber, said fraction being selected among the group consisting of the intervals 0.9-0.1; 0.8-0.2; 0.7-0.3; 0.6-0.4 and 0.55-0.45. Appropriate selection of this fraction may ensure high probability of a therapeutically effective amount of drug reaching the fine receptors of the lungs.

The invention may further concerns a device, wherein the gas containment chamber has a volume with dimensions of the same order as the dimensions of the vent chamber inside the gas canister. Preliminary experiments indicate that such dimensions are appropriate to achieve a relatively large proportion of drug comes in contact with the fine receptors of the lungs.

The invention may further concerns a device, wherein the gas containment chamber has a volume larger than a volume selected among the group consisting of 0.1 cm$^3$; 0.2 cm$^3$; 0.3 cm$^3$; 0.4 cm$^3$; 0.5 cm$^3$; 0.6 cm$^3$; 0.7 cm$^3$; 0.8 cm$^3$; 0.9 cm$^3$ and 1 cm$^3$. Preliminary experiments indicate that such dimensions are appropriate to achieve a relatively large proportion of drug comes in contact with the fine receptors of the lungs.

The invention may further concerns a device, wherein the gas containment chamber has a volume smaller than a volume selected among the group consisting of 100 cm$^3$; 50 cm$^3$; 25 cm$^3$; 10 cm$^3$; 5 cm$^3$; 3 cm$^3$; 2 cm$^3$; 1.7 cm$^3$ and 1.5 cm$^3$.

The invention may further concerns a device, wherein the gas containment chamber has a volume selected among the group consisting of the ranges 0.1-100 cm$^3$; 0.2-50 cm$^3$; 0.3-25 cm$^3$; 0.4-10 cm$^3$; 0.5-5 cm$^3$; 0.6-3 cm$^3$; 0.7-2 cm$^3$; 0.9-1.7 cm$^3$ and 1-1.5 cm$^3$.

Preliminary experiments indicate that the dimensions mentioned above are appropriate to achieve a relatively large proportion of drug comes in contact with the fine receptors of the lungs.

The invention may further concerns a device, provided with removable cover means for covering said outlet valve, when said device is not used for inhalation. This aspect may provide protection of the outlet valve, when the device is not being used.

Drawings

Short Description of the Figures

Fig. I is a schematic diagram of an embodiment of the present invention.
Fig. 2 is a scale drawing of a cross section of a preferred embodiment of the present invention.
Fig. 3 is a scale drawing seen from the outside of an emodiment of the present device, inter alia depicting the gas canister covering part.

Fig. 4 is a scale drawing of a part of the cross section shown in Fig. 2.

Fig. 5 is a scale drawing of a part of the cross section shown in Fig. 2.

Fig. 6 is a section of the scale drawing of Fig. 2.

List of Reference Numerals

Fig. 1

| 1 | device |
|---|---|
| 10 | gas container |
| 12 | wall (of gas container) |
| 14 | gas containment chamber |
| 16 | inlet (of gas container) |
| 18 | outlet |
| 20 | outlet valve |
| 22 | mouthpiece |
| 24 | exhalation valve |
| 26 | receiving means |
| 28 | spacer |
| 30 | valve pin |
| 32 | spring means |
| 34 | inhalation opening |
| 36 | spacing means |
| 38 | outer wall |
| 40 | opening |
| 42 | outer cover |
| 44 | clip |
| 46 | elongated opening |
| 48 | activation pin |
| 50 | sliding means |
| 54 | gas canister holding means |
| 56 | spring attachment means |
| 96 | nozzle (of gas canister) [not part of the invention] |
| 98 | gas canister [not part of the invention] |

Fig. 2-6

| 201 | device |
|---|---|
| 210 | gas container |
| 212 | wall (of gas container) |
| 214 | gas containment chamber |
| 216 | inlet (of gas container) |
| 218 | outlet |
| 220 | outlet valve |
| 222 | mouthpiece |
| 224 | exhalation valve |
| 226 | receiving means |
| 228 | spacer |
| 230 | valve pin |
| 232 | spring means |
| 234 | inhalation opening |
| 236 | spacing means |
| 238 | outer wall |
| 240 | opening |
| 241 | opening |

| 242 | outer cover |
|---|---|
| 244 | clip |
| 246 | elongated opening |
| 248 | activation pin |
| 250 | sliding means |
| 252 | cover means |
| 254 | gas canister holding means |
| 256 | spring attachment means |
| 260 | O-ring |
| 262 | sealing means |
| 264 | connecting part |
| 296 | nozzle (of gas canister) [not part of the invention] |
| 298 | gas canister [not part of the invention] |

Detailed Description of the Figures

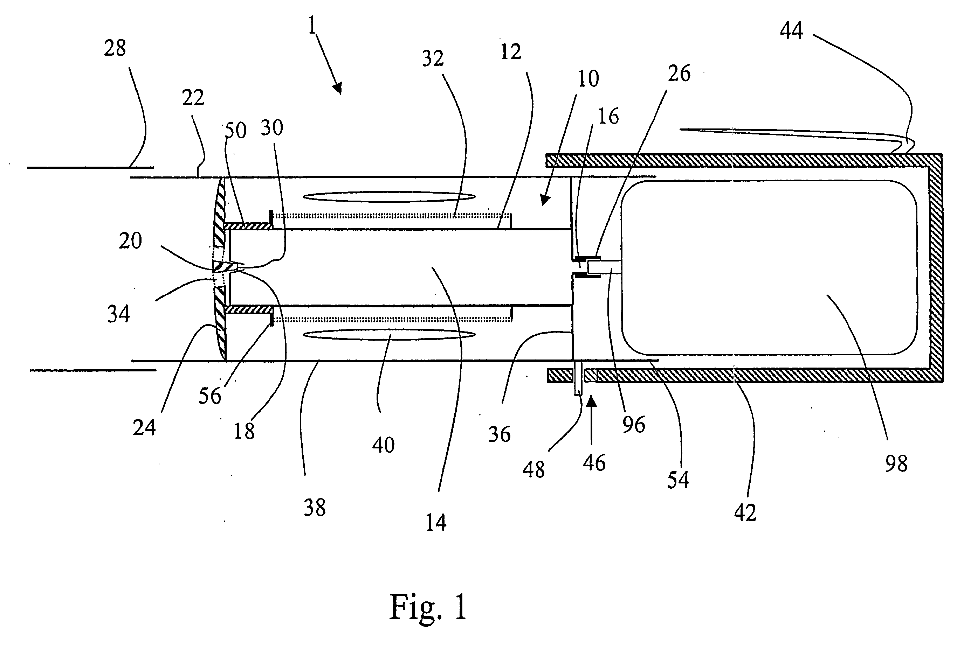

Fig. 1 is a schematic diagram of an embodiment of a device 1. The device 1, adapted for inhalation of drug from a gas canister 98 by a person suffering from asthma or bronchospasm, comprises a gas container 10 with at least one wall 12. The wall 12 surronds a gas containment chamber 14. The gas container 10 further has an inlet 16, adapted for connection to an outlet or nozzle 96 of the gas canister 98. The gas container 10 is provided with an outlet 18 with an outlet valve 20. The gas containment chamber 14 may be filled with gas on activation of the gas canister 98. The person may activate the outlet valve 20 on inhalation, whereby inhalation of the gas from the gas containment chamber 14 is possible. The mouthpiece 22, connected to the device 1, allows the person to inhale with the mouth directly from the device. The device 1 is further provided with a removable spacer 28 for said mouthpiece 22, whereby the mouthpiece 22 is effectively prolonged. The device has an exhalation valve 24, which is in flow communication with the mouthpiece. The person may exhale through the device, without loss of gas from the gas containment chamber 14. The inlet 16 is connected via receiving means 26 to the nozzle 96 of the gas canister 98. The receiving means 26 may be replaced, inter alia for easy adjustment of the device 1 to different types of gas canisters. Further the device 1 has gas canister holding means 54, for secure mounting of the gas canister. The outlet valve comprises a valve pin 30, spring means 32 and spring attachment means 56. The spring means 32 are connected to the gas container 10 and via the spring attachment means 56 to the valve pin 30. The spring means 32 force the valve pin 30 to close the outlet valve 20 except on inhalation, and allows the outlet valve 20 to open upon inhalation. The device 1 has an outer wall 38 and spacing means 36 for connecting the gas container 10 with the outer wall 38. The outlet valve 20 comprises the exhalation valve 24 with a resilient flap valve. The circumference of the resilient flap valve is in close proximity with the outer wall 38. The resilient flap valve has at least one inhalation opening 34 close to the center. This inhalation opening 34 allows gas to pass from the gas containment chamber to the patient upon inhalation. The device 1 has sliding means 50 around the wall 12 of the gas container 10. The sliding means can slide with respect to the outer surface of the wall 12 of the gas container 10, and is connected to the outlet valve 20, whereby the outlet valve will be held in place upon exhalation and inhalation. The outer wall 38 is shaped as a tube with a diameter substantially equal to the diameter of the gas canister. The outer wall 38 surrounds said inlet and said outlet of the gas container 10, and is connected to the gas container by the spacing means 36, as well as to the mouthpiece 22. The outer wall 38 is provided with a number of openings 40, situated between said inlet and said outlet of said gas container, whereby gas may escape upon exhalation. The device is further provided with an outer cover 42, which covers the gas canister. On the outside of the outer cover 42 is placed a clip 44, for fixation of the device 1 to clothes. The outer cover 42 has an elongated opening 46 through which a pin 48, attached to the device 1, protrudes. This makes it possible to effectuate activation of the gas canister 98 by rotation of the outer cover 42 with respect to the rest of the device 1. The outer surfaces of the device are generally cylinder shaped, which means the device mimics a normal thick pen.

Best Mode

Below, an especially preferred embodiment of the present invention will be described.

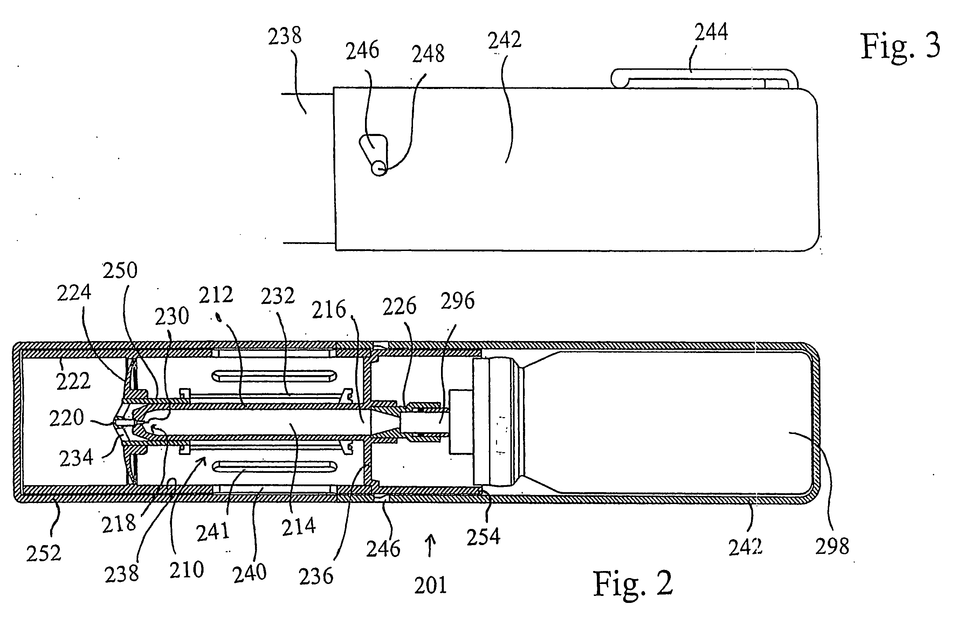

Fig. 2 is a scale drawing of a cross section of a device 201. The device 201, adapted for inhalation of a drug from a gas canister 298 by a person suffering from asthma or bronchospasm, comprises a gas container 210 with at least one wall 212. The wall 212 surrounds a gas containment chamber 214. The gas container 210 further has an inlet 216, adapted for connection to an outlet or nozzle 296 of the gas canister 298. The gas container 210 is provided with an outlet 218 with an outlet valve 220. The gas containment chamber 214 may be filled with gas on activation of the gas canister 298. The person may activate the outlet valve 220 on inhalation, whereby inhalation of the gas from the gas containment chamber 214 is possible. The mouthpiece 222, connected to the device 201, allows the person to inhale with the mouth directly from the device. The device 201 is provided with a removable spacer 228 for the mouthpiece 222, whereby the mouthpiece 222 is effectively prolonged. The device has an exhalation valve 224, which is in flow communication with the mouthpiece. The person may exhale through the device without loss of gas from the gas containment chamber 214. The inlet 216 is connected via receiving means 226 to the nozzle 296 of the gas canister 298. The receiving means 226 may be replaced, inter alia for easy adjustment of the device 201 to different types of gas canisters. Further the device 201 has gas canister holding means 254, for secure mounting of the gas canister. The outlet valve comprises a valve pin 230, spring means 232 and spring attachment means 256. The spring means 232 are connected to the gas container 210 and via the spring attachment means 256 to the valve pin 230. The spring means 232 force the valve pin 230 to close the outlet valve 220 except on inhalation, and allows the outlet valve 220 to open upon inhalation. The device 201 has an outer wall 238 and spacing means 236 for connecting the gas container 210 with the outer wall 238. The outlet valve 220 comprises the exhalation valve 224 with a resilient flap valve. The circumference of the resilient flap valve is in close proximity with the outer wall 238. The resilient flap valve has at least one inhalation opening 234 close to the center. This inhalation opening 234 allows gas to pass from the gas containment chamber to the patient upon inhalation. The device 201 has sliding means 250 around the wall 212 of the gas container 210. The sliding means can slide with respect to the outer surface of the wall 212 of the gas container 210, and is connected to the outlet valve 220, whereby the outlet valve will be held in place upon exhalation and inhalation. The outer wall 238 is shaped as a tube with a diameter substantially equal to the diameter of the gas canister. The outer wall 238 surrounds said inlet and said outlet of the gas container 210, and is connected to the gas container by the spacing means 236, as well as to the mouthpiece 222. The outer wall 238 is provided with a number of openings 240 and 241, situated between said inlet and said outlet of said gas container, whereby gas may escape upon exhalation. The device is further provided with an outer cover 242, which covers the gas canister. When the device is not used for inhalation and/or exhalation, it is provided with removable cover means 252. The cover means protects the outer valve 220 and covers the openings 240 and 241, such that dirt does not enter the device 201.

Generally, all parts of the device are made from plastic. However, for certain parts, other materials are preferred. For the valve pin 230 titanium is preferred, for the spring means 232 rubber bands are preferred, for the resilient flap valve of the exhalation valve 224 silicone is preferred, and for the clip 244 a resilient material such as metal is preferred. The length of the depicted device is about 170 mm.

The strength of the spring means, i.e. the rubber bands, is easily adjusted by choosing it such that a mass of 5 g, hanging freely from the device, is adequate for activating the outlet valve.

The area, $A_1$, of the tip of the pin is less than 0.3 mm$^2$, while the area, $A_2$, of the resilient flap is about 550 mm$^2$. Thus, by appropriate choice of spring means, very high values of OPV parameter may be obtained, e.g. 1500.

Fig. 3 is a scale drawing seen from the outside of the device. The outer cover 242 conceals the gas canister and part of the outer wall 238. On the outside of the outer cover 242 is placed a clip 244, for fixation of the device to clothes. The outer surfaces of the device are generally cylinder shaped, which means the device mimics a normal thick pen. The outer cover 242 has a triangular shaped elongated opening 246 through which an activation pin 248, attached to the device 201, protrudes. The figure shows the activation pin 248 in the activation position, where at least one dosis of drug has been released from the gas canister. By turning the outer cover 242 with respect to the outer wall 238 back and forth, additional doses may be released from the gas canister, because the activation pin 248 engages the elongated opening 246. It is thus possible to effectuate activation of the gas canister 298 by rotation of the outer cover 242 with respect to the outer wall 238.

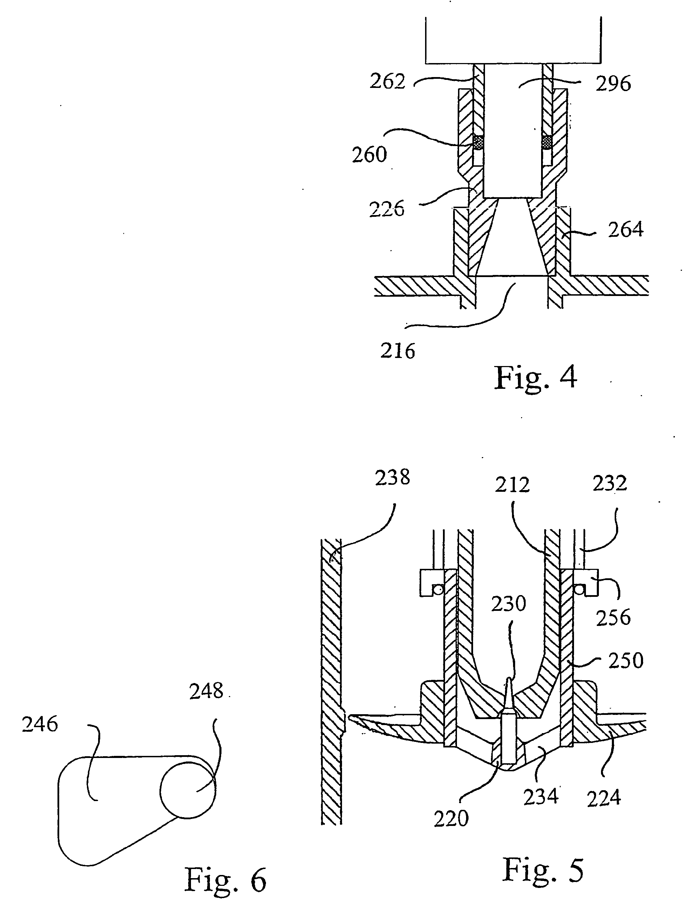

Fig. 4 is a scale drawing of a section of the cross section shown in Fig. 2, which depicts the construction around the inlet 216 of the gas container. The inlet 216 is in flow communication with connecting part 264, on which is mounted receiving means 226. Inside the receiving means 226 is the nozzle 296 of the gas canister. Sealing means 262 is provided for sealing between the receiving means 226 and the nozzle 296. An O-ring 260 is also provided at the end of the sealing means 262 for improving the sealing and softening the impact of sealing means 262 engaging receiving means 226.

Fig. 5 is a scale drawing of a section of the cross section shown in Fig. 2, which depicts the outlet valve 220. The outlet valve 220 is surrounded by the outer wall 238, and is mounted on sliding means 250, whereby the outlet valve 220 moves with respect to the wall 212 of the gas container. The valve pin 230, mounted in the center of the outlet valve 220, closes the outlet. The exhalation valve 224 is actuated by inhalation and exhalation of the patient, and forces, together with the spring means 232, fixed to the spring attachment means 256, the outlet valve 220 to open upon inhalation and close upon exhalation. The inhalation opening 234 allows gas containing an active drug to pass to the patient upon inhalation.

Fig. 6 shows a detail from the scale drawing of Fig. 3, depicting the triangular shape of the elongated opening 246, with an activation pin 248 sticking through. This will help a person with empaired vision in ascertaining the activation state of the gas canister, as the position of the activation pin 248 may be felt with a finger. In the preferred embodiment, the device is provided with two such activations pins, opposite each other, on each side of the device.

In order to mount the outer cover 242 for covering the gas canister 298, the outer cover 242, made of resilient plastic, is preferably pressed slightly close to its open end, thereby changing the form of the open end from having a circular cross section to being slightly oval, whereby the outer cover 242 may slide above the activation pin 248, and be locked in place upon release of the pressure.

As this preferred embodiment is only intended to be used once and then discarded, it is designed to make it cumbersome to exchange the gas canister, after the gas canister has been mounted.

The foregoing description is only intended to elucidate, not limit, the present invention.

**Claims**

1. A device (1) for the inhalation of at least one drug, from a gas canister (98) having a nozzle (96), by a person suffering from asthma or bronchospasm, said device (1) comprising:

   a gas container (10) having at least one wall (12), said wall (12) surrounding a gas containment chamber (14) adapted to contain gas at a pressure larger than atmospheric pressure, said gas container (10) further having an inlet (16) adapted for connection to the nozzle (96) of the gas canister (98), said gas container (10) further having an outlet (18) with an outlet valve (20) adapted to allow the person to inhale gas from said device (1) through said outlet (18);

   wherein said outlet valve (20) is adapted to be activated by the inhalation of the person independent of the activation of the gas canister (98);
   **characterized by** said device (1) comprising sliding means (50, 250) connected to said outlet valve (20), said sliding means (50, 250) being adapted to slide with respect to said wall (12) of said gas container (10) upon activation of said outlet valve (20).

2. The device (1) according to claim 1 further comprising a mouthpiece (22) adapted to allow the person to inhale from said mouthpiece (22), said mouthpiece (22) being in flow communication with said outlet valve (20).

3. The device (1) according to claim 2, further provided with a removable spacer (28) for said mouthpiece (22), said spacer (28) allowing prolonging said mouthpiece (22) on mounting.

4. The device (1) according to claim 2 further comprising an exhalation valve (24); wherein said mouthpiece (22) is in flow communication with said exhalation valve (24), allowing the person to exhale through the mouthpiece (22).

5. The device (1) according to any of the preceding claims, wherein said inlet (16) is provided with receiving means (26) being connectable to said inlet (16) of said gas container (10), wherein said receiving means (26) is adapted to receive the nozzle (96) of the gas canister (98).

6. The device (1) according to any of the preceding claims, having gas canister holding means (54) connected to said gas container (10), said gas canister holding means (54) being adapted to receiving and holding the gas canister (98).

7. The device (1) according to any of the preceding claims;
   wherein the atmosphere surrounding said device (1) has a pressure, $P_{atm}$; said outlet valve (20) experiences an inner pressure from said gas containment chamber (14) before activation of said outlet valve, $P_{ca}$; and said outlet valve (20) experiences an outer pressure before activation of said outlet valve (20), $P_{ma}$;
   wherein said outlet valve (20) is adapted to have an outlet valve parameter, $OVP$, larger than 1; said outlet valve parameter, $OVP$, being defined by the condition for activation, $B$, of said outlet valve (20):

$$B: \quad | P_{ca} - P_{atm} | - OVP | P_{atm} - P_{ma} | < 0 \text{ atm;}$$

   such that said outlet valve (20) of said container (10) is activated when said condition for activation, $B$, is fulfilled.

8. The device (1) according to claim 7, wherein said outlet valve (20) is adapted to have an outlet valve parameter, $OVP$, larger than a number selected among the group consisting of 20; 15; 10; 8; 6; 5; 4; 3; 2.5; 2; 1.5; 1.4; 1.3; 1.2 and 1.1.

9. The device (1) according to any of the preceding claims, wherein said outlet valve (20) comprises a valve pin (30),

spring means (32) and spring attachment means; said spring means (32) being connected to said gas container (10), and via said spring attachment means being connected to and forcing said valve pin (30) to close said outlet valve (20) except on inhalation, and allowing said outlet valve (20) to open upon inhalation.

10. The device (1) according to any of the preceding claims, wherein the gas in said gas containment chamber (14) exerts a force on said outlet valve (20) on a first area, $A_1$, and the outer pressure exerts a force on the outlet valve (20) on a second area, $A_2$, said first area being smaller than said second area; $A_1 < A_2$.

11. The device (1) according to claim 10, wherein the ratio, $A_2/A_1$, between said second area, $A_2$, and said first area, $A_1$, is larger than a number selected among the group consisting of 5000; 2000; 1500; 1000; 500; 300; 100; 50; 30; 20; 10; 5; 4; 3; 2 and 1.5.

12. The device (1) according to any of the preceding claims, said device (1) comprising an outer wall (38) and spacing means (36) for connecting said gas container (10) with said outer wall (38); wherein said outlet valve (20) comprises an exhalation valve (24) having a resilient flap valve with a circumference in close proximity to said outer wall (38), said resilient flap valve being adapted to allow air to be exhaled without activation of said outlet valve (20), and allowing activation of said outlet valve (20) upon inhalation; and said resilient flap valve having an inhalation opening through which gas from said gas containment chamber (14) may pass, allowing gas to flow from the gas containment chamber (14) to the patient upon inhalation.

13. The device (1) according to claim 12, wherein the force necessary to bend said resilient flap valve in direction towards the person is larger than the force necessary to bend said resilient flap valve in direction away from the person.

14. The device (1) according to claim 12 or 13, wherein at least part of the resilient flap is convex with respect to the person.

15. The device (1) according to claim 12, 13 or 14, wherein at least part of the resilient flap is concave with respect to the direction of the gas canister (98).

16. The device (1) according to any of the claims 12 - 15, wherein said resilient flap valve allows air to flow substantially freely on exhalation, while only allowing a limited stream of air to pass between said circumference of said resilient flap valve and the inner surface of said outer wall (38) upon inhalation.

17. The device (1) according to any of the preceding claims, wherein said sliding means (50, 250) surrounds said outer surface of said wall (12) of said gas container (10) completely, in a substantially air tight way.

18. The device (1) according to any of the preceding claims, said device (1) comprising an outer wall (38) shaped as a tube with a diameter substantially equal to the diameter of the gas canister (98), said outer wall (38) surrounding said inlet (16) and said outlet (18) of said gas container (10), and said device (1) comprising spacing means (36) for connecting said gas container (10) with said outer wall (38); wherein said outer wall (38) is connected to said mouthpiece (22).

19. The device (1) according to claim 18, wherein said outer wall (38) further surrounds at least part of the gas canister (98).

20. The device (1) according to claim 18 or 19, wherein said outer wall (38) is provided with at least one opening (40) situated between said inlet (16) and said outlet (18) of said gas container (10), for allowing air to be let out of the device (1) upon exhalation.

21. The device (1) according to any of the preceding claims, said device (1) comprising an outer cover (42), said outer cover (42) covering at least part of the bottom part of the gas canister (98), wherein said cover (42) is adapted as to closely fit the gas canister (98) and allow movement of said outer cover (42) with respect to said gas container (10), so that the gas container (10) may be activated by movement of said cover (42).

22. The device (1) according to claim 21, wherein said outer cover (42) is provided with a clip (44) allowing fixation of the device (1) to clothes.

23. The device (1) according to claim 21 or 22, wherein the gas canister (98) is activated by rotation of said outer cover (42) with respect to said gas container (10).

**24.** The device (1) according to claim 23, wherein the outer cover (42) comprises an elongated opening (46), said elongated opening (46) having at least a part slanted with respect to the longitudinal direction of the gas canister (98), and said device (1) further comprises an activation pin (48) connected to said gas container (10), said pin (48) engaging said elongated opening (46) such that rotation of said outer cover (42) with respect to said gas container (10) activates said gas canister (98).

**25.** The device (1) according to any of the preceding claims, wherein the outer surfaces of said device (1) is shaped as a cylinder.

**26.** The device (1) according to any of the preceding claims, wherein the gas containment chamber (14) is adapted to contain gas at a pressure larger than a pressure selected among the group consisting of 1.05; 1.1; 1.2; 1.3; 1.5; 1,6; 1.7; 1.8; 1.9; 2; 2.5 and 3 atm.

**27.** The device (1) according to any of the preceding claims, wherein the gas containment chamber (14) is adapted to contain gas at a pressure smaller than a pressure selected among the group consisting of 4; 3; 2.5; 2; 1.5; 1.4; 1.3; 1.2 and 1.1 atm.

**28.** The device (1) according to any of the preceding claims, wherein the gas containment chamber (14) has dimensions which reduces the speed of gas leaving the chamber (14) to a fraction of the speed of gas entering the chamber (14).

**29.** The device (1) according to any of the preceding claims, wherein the gas containment chamber (14) has dimensions which reduces the speed of gas leaving the chamber (14) to a fraction of the speed of gas entering the chamber (14), said fraction being smaller than a number selected among the group consisting of 0.9; 0.8; 0.7; 0.6 and 0.55.

**30.** The device (1) according to any of the preceding claims, wherein the gas containment chamber (14) has dimensions which reduces the speed of gas leaving the chamber (14) to a fraction of the speed of gas entering the chamber (14), said fraction being larger than a number selected among the group consisting of 0.1; 0.2; 0.3; 0.4 and 0.45.

**31.** The device (1) according to any of the preceding claims, wherein the gas containment chamber (14) has dimensions which reduces the speed of gas leaving the chamber (14) to a fraction of the speed of gas entering the chamber (14), said fraction being selected among the group consisting of the intervals 0.9-0.1; 0.8-0.2; 0.7-0.3; 0.6-0.4 and 0.55-0.45.

**32.** The device (1) according to any of the preceding claims, wherein the gas containment chamber (14) has a volume with dimensions of the same order as the dimensions of the vent chamber inside the gas canister (98) .

**33.** The device (1) according to any of the preceding claims, wherein the gas containment chamber (14) has a volume larger than a volume selected among the group consisting of 0.1 cm$^3$; 0.2 cm$^3$; 0.3 cm$^3$; 0.4 cm$^3$; 0.5 cm$^3$; 0.6 cm$^3$; 0.7 cm$^3$: 0.8 cm$^3$; 0.9 cm$^3$ and 1 cm$^3$.

**34.** The device (1) according to any of the preceding claims, wherein the gas containment chamber (14) has a volume smaller than a volume selected among the group consisting of 100 cm$^3$; 50 cm$^3$; 25 cm$^3$; 10 cm$^3$; 5 cm$^3$: 3 cm$^3$; 2 cm$^3$; 1.7 cm$^3$ and 1.5 cm$^3$.

**35.** The device (1) according to any of the preceding claims, wherein the gas containment chamber (14) has a volume selected among the group consisting of the ranges 0-1-100 cm$^3$; 0.2-50 cm$^3$; 0.3-25 cm$^3$; 0.4-10 cm$^3$; 0.5-5 cm$^3$; 0.6-3 cm$^3$; 0.7-2 cm$^3$; 0.9-1.7 cm$^3$ and 1-1.5 cm$^3$.

**36.** The device (1) according to any of the preceding claims, provided with removable cover means for covering said outlet valve (20), when said device (1) is not used for inhalation.

**Patentansprüche**

**1.** Vorrichtung (1) für die Inhalation von mindestens einem Medikament aus einer Gasflasche (98) mit einer Düse (96) durch eine Person, die an Asthma oder Bronchospasmen leidet, wobei die Vorrichtung (1) umfasst:

einen Gasbehälter (10) mit mindestens einer Wand (12),

wobei die Wand (12) eine Gasdruckkammer (14) umgibt, die geeignet ist, ein Gas unter einem Druck von mehr als dem Atomsphärendruck zu enthalten, wobei der Gasbehälter (10) weiter einen Einlass (16) besitzt, der zur Verbindung mit der Düse (96) der Gasflasche (98) geeignet ist, wobei der Gasbehälter (10) weiter einen Auslass (18) mit einem Auslassventil (20) besitzt, das geeignet ist, es der Person zu erlauben, Gas aus der Vorrichtung (1) durch den Auslass (18) zu inhalieren;

wobei das Auslassventil (20) geeignet ist, durch die Inhalation der Person unabhängig von der Aktivierung der Gasflasche (98) aktiviert zu werden;

**dadurch gekennzeichnet, dass** die Vorrichtung (1) eine mit dem Auslassventil (20) verbundene Gleitvorrichtung (50, 250) umfasst, wobei die Gleitvorrichtung (50, 250) geeignet ist, bei Aktivierung des Auslassventils (20) in Bezug auf die Wand (12) der Gasbehälters (10) zu gleiten.

2. Vorrichtung (1) nach Anspruch 1, weiter ein Mundstück (22) umfassend, das geeignet ist, es der Person zu erlauben, aus dem Mundstück (22) zu inhalieren, wobei das Mundstück (22) in Fließverbindung mit dem Auslassventil (20) steht.

3. Vorrichtung (1) nach Anspruch 2, die weiter mit einem entfernbaren Distanzstück (28) für das Mundstück (22) versehen ist, wobei das Distanzstück (28) beim Anbringen die Verlängerung des Mundstücks (22) erlaubt.

4. Vorrichtung (1) nach Anspruch 2, weiter ein Ausatemventil (24) umfassend; wobei das Mundstück (22) in Fließverbindung mit dem Ausatemventil (24) steht und es so der Person erlaubt, durch das Mundstück (22) auszuatmen.

5. Vorrichtung (1) nach einem der vorangehenden Ansprüche, wobei der Einlass (16) mit einem Aufnahmemittel (26) versehen ist, das mit dem Einlass (16) des Gasbehälters (10) verbindbar ist, wobei das Aufnahmemittel (26) geeignet ist, die Düse (96) der Gasflasche (98) aufzunehmen.

6. Vorrichtung (1) nach einem der vorangehenden Ansprüche mit einer Gasflaschenhaltevorrichtung (54), die mit dem Gasbehälter (10) verbunden ist, wobei die Gasflaschenhaltevorrichtung (54) geeignet ist, die Gasflasche (98) aufzunehmen und zu halten.

7. Vorrichtung (1) nach einem der vorangehenden Ansprüche, wobei die die Vorrichtung (1) umgebene Atmosphäre einen Druck ($P_{atm}$) besitzt; das Auslassventil (20) vor der Aktivierung des Auslassventils aus der Gasdruckkammer (14) einen Innendruck ($P_{ca}$) erfährt; und das Auslassventil vor Aktivierung des Auslassventils (20) einen Außendruck ($P_{ma}$) erfährt;

wobei das Auslassventil (20) geeignet ist, einen Auslassventilparameter (OVP) von größer als 1 zu besitzen, wobei der Auslassventilparameter (OVP) durch die Bedingung zur Aktivierung (B) des Auslassventils (20) definiert ist:

$$B: \quad | P_{ca} - P_{atm} | - OVP | P_{atm} - P_{ma} | < 0 \text{ atm};$$

so dass das Auslassventil (20) des Behälters (10) aktiviert wird, wenn die Bedingung zur Aktivierung (B) erfüllt ist.

8. Vorrichtung (1) nach Anspruch 7, wobei das Auslassventil (20) geeignet ist, einen Auslassventilparameter (OVP) größer als eine Zahl zu besitzen, die aus der Gruppe ausgewählt ist, welche aus 20; 15; 10; 8; 6; 5; 4; 3; 2, 5; 2; 1, 5; 1, 4; 1, 3; 1,2 und 1,1 besteht.

9. Vorrichtung (1) nach einem der vorangehenden Ansprüche, wobei das Auslassventil (20) einen Ventilstift (30), eine Federvorrichtung (32) und eine Federbefestigungsvorrichtung umfasst; wobei die Federvorrichtung (32) mit dem Gasbehälter (10) verbunden ist und über die Federbefestigungsvorrichtung mit dem Ventilstift (30) verbunden ist und ihn so vorspannt, dass das Auslassventil (20) außer bei der Inhalation geschlossen ist, und es erlaubt, dass das Auslassventil (20) bei Inhalation offen ist.

10. Vorrichtung (1) nach einem der vorangehenden Ansprüche, wobei das Gas in der Gasdruckkammer (14) eine Kraft auf das Auslassventil (20) an einer ersten Fläche ($A_1$) ausübt und der Außendruck eine Kraft auf das Auslassventil (20) auf einer zweiten Fläche ($A_2$) ausübt, wobei die erste Fläche kleiner als die zweite Fläche ist; $A_1 < A_2$.

11. Vorrichtung (1) nach Anspruch 10, wobei das Verhältnis $A_2/A_1$ zwischen der zweiten Fläche $A_2$ und der ersten Fläche $A_1$ größer als eine Zahl ist, die aus der aus 5000; 2000; 1500; 1000; 500; 300; 100; 50; 30; 20; 10; 5; 4; 3;

2 und 1,5 bestehenden Gruppe ausgewählt ist.

12. Vorrichtung (1) nach einem der vorangehenden Ansprüche, wobei die Vorrichtung (1) eine Außenwand (38) und eine Beabstandungsvorrichtung (36) zur Verbindung des Gasbehälters (10) mit der Außenwand (38) umfasst; wobei das Auslassventil (20) ein Ausatmenventil (24) mit einem elastischen Klappenventil mit einem Umfang in enger Nähe zur Außenwand (38) umfasst, wobei das elastische Klappenventil geeignet ist, das Ausatmen von Luft ohne Aktivierung des Auslassventils (20) zu erlauben und die Aktivierung des Auslassventils (20) bei Inhalation erlaubt; und wobei das elastische Klappenventil eine Inhalationsöffnung besitzt, durch welche Gas aus der Gasdruckkammer (14) durchtreten kann und es so dem Gas erlaubt, aus der Gasdruckkammer (14) bei Inhalation zum Patienten zu fließen.

13. Vorrichtung (1) nach Anspruch 12, wobei die Kraft, die notwendig ist, um das elastische Klappenventil in Richtung zur Person zu biegen, größer ist als die Kraft, die notwendig ist, um das elastische Klappenventil in Richtung von der Person weg zu biegen.

14. Vorrichtung (1) nach Anspruch 12 oder 13, wobei mindestens ein Teil der elastischen Klappe in Bezug auf die Person konvex ist.

15. Vorrichtung (1) nach Anspruch 12, 13 oder 14, wobei mindestens ein Teil der elastischen Klappe in Bezug auf die Richtung der Gasflasche (98) konkav ist.

16. Vorrichtung (1) nach einem der Ansprüche 12-15, wobei das elastische Klappenventil Luft beim Ausatmen im Wesentlichen frei strömen lässt, während es beim Einatmen lediglich eine begrenzte Luftströmung zwischen dem Umfang des elastischen Klappenventils und der inneren Oberfläche der äußeren Wand (38) durchtreten lässt.

17. Vorrichtung (1) nach einem der vorangehenden Ansprüche, wobei die Gleitvorrichtung (50, 250) die äußere Oberfläche der Wand (12) des Gasbehälters (10) vollständig auf im Wesentlichen luftdichte Weise umgibt.

18. Vorrichtung (1) nach einem der vorangehenden Ansprüche, wobei die Vorrichtung (1) eine Außenwand (38) umfasst, die geformt ist wie ein Rohr mit einem Durchmesser, der im Wesentlichen gleich dem Durchmesser der Gasflasche (98) ist, wobei die äußere Wand (38) den Einlass (16) und den Auslass (18) des Gasbehälters (10) umgibt, und die Vorrichtung (1) Beabstandungsmittel (36) umfasst, um den Gasbehälter (10) mit der äußeren Wand (38) zu verbinden; wobei die äußere Wand (38) mit dem Mundstück (22) verbunden ist.

19. Vorrichtung (1) nach Anspruch 18, wobei die äußere Wand (38) weiter mindestens einen Teil der Gasflasche (98) umgibt.

20. Vorrichtung (1) nach Anspruch 18 oder 19, wobei die äußere Wand (38) mit mindestens einer Öffnung (40) versehen ist, die zwischen dem Einlass (16) und dem Auslass (18) des Gasbehälters (10) angeordnet ist, um beim Ausatmen Luft aus der Vorrichtung (1) herauszulassen.

21. Vorrichtung (1) nach einem der vorangehenden Ansprüche, wobei die Vorrichtung (1) eine äußere Abdeckung (42) umfasst, wobei die äußere Abdeckung (42) mindestens einen Teil des unteren Teils der Gasflasche (98) bedeckt, wobei die Abdeckung (42) geeignet ist, eng auf die Gasflasche (98) zu passen und die Bewegung der äußeren Abdeckung (42) in Bezug auf den Gasbehälter (10) zu erlauben, so dass der Gasbehälter (10) durch Bewegung der Abdeckung (42) betätigt werden kann.

22. Vorrichtung 1 nach Anspruch 21, wobei die äußere Abdeckung (42) mit einem Clip (44) versehen ist, der die Befestigung der Vorrichtung (1) an Kleidung erlaubt.

23. Vorrichtung 1 nach Anspruch 21 oder 22, wobei die Gasflasche (98) durch Drehung der äußeren Abdeckung (42) in Bezug auf den Gasbehälter (10) aktiviert wird.

24. Vorrichtung (1) nach Anspruch 23, wobei die äußere Abdeckung (42) eine längliche Öffnung (46) umfasst, wobei die längliche Öffnung (46) mindestens einen in Bezug auf die Längsrichtung der Gasflasche (98) abgeschrägten Teil aufweist und die Vorrichtung (1) weiter einen Betätigungsstift (48) umfasst, der mit dem Gasbehälter (10) verbunden ist, wobei der Stift (48) mit der länglichen Öffnung (46) so eingreift, dass eine Drehung der äußeren Abdeckung (42) in Bezug auf den Gasbehälter (10) die Gasflasche (98) aktiviert.

25. Vorrichtung (1) nach einem der vorangehenden Ansprüche, wobei die äußere Oberfläche der Vorrichtung (1) als Zylinder geformt ist.

26. Vorrichtung (1) nach einem der vorangehenden Ansprüche, wobei die Gasdruckkammer (14) geeignet ist, um ein Gas unter einem Druck zu enthalten, der größer als ein Druck ist, welcher aus der aus 1,05; 1,1; 1,2; 1,3; 1,5; 1,6; 1,7; 1,8; 1,9; 2; 2,5 und 3 atm bestehenden Gruppe ausgewählt ist.

27. Vorrichtung (1) nach einem der vorangehenden Ansprüche, wobei die Gasdruckkammer (14) geeignet ist, um Gas unter einem Druck zu enthalten, der kleiner als ein Druck ist, der aus der aus 4; 3; 2,5; 2; 1,5; 1,4; 1,3; 1,2 und 1,1 atm bestehenden Gruppe ausgewählt ist.

28. Vorrichtung (1) nach einem der vorangehenden Ansprüche, wobei die Gasdruckkammer (14) Abmessungen besitzt, die die Geschwindigkeit des die Kammer (14) verlassenden Gases auf einen Bruchteil der Geschwindigkeit des in die Kammer eintretenden Gases reduzieren.

29. Vorrichtung (1) nach einem der vorangehenden Ansprüche, wobei die Gasdruckkammer (14) Abmessungen besitzt, welche die Geschwindigkeit des die Kammer (14) verlassenden Gases auf einen Bruchteil der Geschwindigkeit des in die Kammer (14) eintretenden Gases reduzieren, wobei der Bruchteil kleiner als eine Zahl ist, die aus der aus 0,9; 0,8; 0,7; 0,6 und 0,55 bestehenden Gruppe ausgewählt ist.

30. Vorrichtung (1) nach einem der vorangehenden Ansprüche, wobei die Gasdruckkammer (14) Abmessungen besitzt, die die Geschwindigkeit des die Kammer (14) verlassenden Gases auf einen Bruchteil der Geschwindigkeit des in die Kammer eintretenden Gases reduzieren, wobei der Bruchteil größer als eine Zahl ist, die aus der aus 0,1; 0,2; 0,3; 0,4 und 0,45 bestehenden Gruppe ausgewählt ist.

31. Vorrichtung (1) nach einem der vorangehenden Ansprüche, wobei die Gasdruckkammer (14) Abmessungen besitzt, welche die Geschwindigkeit des die Kammer (14) verlassenen Gases auf einem Bruchteil der Geschwindigkeit des in die Kammer (14) eintretenden Gases reduzieren, wobei der Bruchteil ausgewählt ist aus der Gruppe, die aus den Intervallen 0,9-0,1; 0,8-0,2; 0,7-0,3; 0,6-0,4; und 0,55- 0,45 besteht.

32. Vorrichtung (1) nach einem der vorangehenden Ansprüche, wobei die Gasdruckkammer (14) ein Volumen mit Abmessungen derselben Größenordnung besitzt, wie die Abmessungen der Entlüftungskammer in der Gasflasche (98).

33. Vorrichtung (1) nach einem der vorangehenden Ansprüche, wobei die Gasdruckkammer (14) ein Volumen besitzt, das größer als ein Volumen ist, das aus der Gruppe ausgewählt ist, die aus $0,1$ cm$^3$; $0,2$ cm$^3$; $0,3$ cm$^3$; $0,4$ cm$^3$; $0,5$ cm$^3$; $0,6$ cm$^3$; $0,7$ cm$^3$; $0,8$ cm$^3$; $0,9$ cm$^3$ und $1$ cm$^3$ besteht.

34. Vorrichtung (1) nach einem der vorangehenden Ansprüche, wobei die Gasdruckkammer (14) ein Volumen besitzt, das kleiner als ein Volumen ist, das aus der Gruppe ausgewählt ist, die aus $100$ cm$^3$; $50$ cm$^3$; $25$ cm$^3$; $10$ cm$^3$; $5$ cm$^3$; $3$ cm$^3$; $2$ cm$^3$; $1,7$ cm$^3$ und $1,5$ cm$^3$ besteht.

35. Vorrichtung (1) nach einem der vorangehenden Ansprüche, wobei die Gasdruckkammer (14) ein Volumen besitzt, das aus der Gruppe ausgewählt ist, die aus den Bereichen $0,1$-$100$ cm$^3$; $0,2$-$50$ cm$^3$; $0,3$-$25$ cm$^3$; $0,4$-$10$ cm$^3$; $0,5$-$5$ cm$^3$; $0,6$-$3$ cm$^3$; $0,7$-$2$ cm$^3$; $0,9$-$1,7$ cm$^3$ und $1$-$1,5$ cm$^3$ besteht.

36. Vorrichtung (1) nach einem der vorangehenden Ansprüche, die mit einer entfernbaren Abdeckvorrichtung zum Abdecken des Auslassventils 20 versehen ist, wenn die Vorrichtung (1) nicht zur Inhalation verwendet wird.

**Revendications**

1. Dispositif (1) pour l'inhalation d'au moins un médicament, à partir d'une cartouche de gaz (98) ayant une buse (96), par une personne souffrant d'asthme ou de bronchospasme, ledit dispositif (1) comprenant:

   un récipient de gaz (10) ayant au moins une paroi (12), ladite paroi (12) entourant une chambre de confinement de gaz (14) adaptée pour contenir un gaz à une pression supérieure à la pression atmosphérique, ledit récipient de gaz (10) ayant en outre une entrée (16) adaptée pour être reliée à la buse (96) de la cartouche de gaz (98), ledit récipient de gaz (10) ayant en outre une sortie (18) avec une soupape de sortie (20) adaptée pour permettre à la personne d'inhaler le gaz à partir dudit dispositif (1) à travers ladite sortie (18) ;

dans lequel ladite soupape de sortie (20) est adaptée pour être activée par l'inhalation de la personne indépendamment de l'activation de la cartouche de gaz (98) ;
**caractérisé en ce que** ledit dispositif (1) comprend un moyen coulissant (50, 250) relié à ladite soupape de sortie (20), ledit moyen coulissant (50, 250) étant adapté pour coulisser par rapport à ladite paroi (12) dudit récipient de gaz (10) lors de l'activation de ladite soupape de sortie (20).

2. Dispositif (1) selon la revendication 1 comprenant en outre une embouchure (22) adaptée pour permettre à la personne d'inhaler à partir de ladite embouchure (22), ladite embouchure (22) étant en communication fluidique avec ladite soupape de sortie (20).

3. Dispositif (1) selon la revendication 2, muni en outre d'un élément d'espacement amovible (28) pour ladite embouchure (22), ledit élément d'espacement (28) permettant le prolongement de ladite embouchure (22) une fois monté.

4. Dispositif (1) selon la revendication 2 comprenant en outre une soupape d'expiration (24) ; dans lequel ladite embouchure (22) est en communication fluidique avec ladite soupape d'expiration (24), permettant à la personne d'expirer à travers l'embouchure (22).

5. Dispositif (1) selon l'une quelconque des revendications précédentes, dans lequel ladite entrée (16) est munie d'un moyen de réception (26) pouvant être relié à ladite entrée (16) dudit récipient de gaz (10), dans lequel ledit moyen de réception (26) est adapté pour recevoir la buse (96) de la cartouche de gaz (98).

6. Dispositif (1) selon l'une quelconque des revendications précédentes, ayant un moyen de maintien de la cartouche de gaz (54) relié audit récipient de gaz (10), ledit moyen de maintien de la cartouche de gaz (54) étant adapté pour recevoir et maintenir la cartouche de gaz (98).

7. Dispositif (1) selon l'une quelconque des revendications précédentes ;
dans lequel l'atmosphère entourant ledit dispositif (1) a une pression, $P_{atm}$; ladite soupape de sortie (20) subissant une pression interne depuis la chambre de confinement de gaz (14) avant l'activation de ladite soupape de sortie, $P_{ca}$; et ladite soupape de sortie (20) subissant une pression externe avant l'activation de ladite soupape de sortie (20), $P_{ma}$ ;
dans lequel ladite soupape de sortie (20) est adaptée pour avoir un paramètre de soupape de sortie, *OVP*, supérieur à 1 ; ledit paramètre de soupape de sortie, *OVP*, étant défini par la condition pour l'activation, *B*, de ladite soupape de sortie (20) :

$$B \; : \quad \left| P_{ca} - P_{atm} \right| - OVP \left| P_{atm} - P_{ma} \right| < 0 \; \text{atm} \; ;$$

de sorte que ladite soupape de sortie (20) dudit récipient (10) soit activée lorsque la condition pour l'activation, *B*, est remplie.

8. Dispositif (1) selon la revendication 7, dans lequel ladite soupape de sortie (20) est adaptée pour avoir un paramètre de soupape de sortie, *OVP*, supérieur à un nombre choisi dans le groupe constitué de 20 ; 15 ; 10 ; 8 ; 6 ; 5 ; 4 ; 3 ; 2,5 ; 2 ; 1,5 ; 1,4 ; 1,3 ; 1,2 et 1,1.

9. Dispositif (1) selon l'une quelconque des revendications précédentes, dans lequel ladite soupape de sortie (20) comprend une goupille de soupape (30), un moyen de ressort (32) et un moyen de fixation à ressort ; ledit moyen de ressort (32) étant relié audit récipient de gaz (10), et par l'intermédiaire dudit moyen de fixation à ressort étant relié à ladite goupille de soupape (30) et forçant cette dernière à fermer ladite soupape de sortie (20) sauf pendant l'inhalation, et permettant à ladite soupape de sortie (20) de s'ouvrir lors de l'inhalation.

10. Dispositif (1) selon l'une quelconque des revendications précédentes, dans lequel le gaz dans ladite chambre de confinement de gaz (14) exerce une force sur ladite soupape de sortie (20) sur une première zone, $A_1$, et la pression externe exerce une force sur la soupape de sortie (20) sur une seconde zone, $A_2$, ladite première zone étant inférieure à ladite seconde zone ; $A_1 < A_2$.

**11.** Dispositif (1) selon la revendication 10, dans lequel le rapport $A_2/A_1$ entre ladite seconde zone, $A_2$, et ladite première zone, $A_1$, est supérieur à un nombre choisi dans le groupe constitué de 5000 ; 2000 ; 1500 ; 1000 ; 500 ; 300 ; 100 ; 50 ; 30 ; 20 ; 10 ; 5 ; 4 ; 3 ; 2 et 1,5.

**12.** Dispositif (1) selon l'une quelconque des revendications précédentes, ledit dispositif (1) comprenant une paroi externe (38) et un moyen d'espacement (36) pour relier ledit récipient de gaz (10) à ladite paroi externe (38) ; dans lequel ladite soupape de sortie (20) comprend une soupape d'expiration (24) ayant une soupape à clapet résilient avec une circonférence à proximité étroite de ladite paroi externe (38), ladite soupape à clapet résilient étant adaptée pour permettre à l'air d'être expiré sans l'activation de ladite soupape de sortie (20), et permettant l'activation de ladite soupape de sortie (20) lors de l'inhalation ; et ladite soupape à clapet résilient ayant une ouverture d'inhalation à travers laquelle le gaz issu de la chambre de confinement de gaz (14) peut passer, permettant au gaz de circuler de la chambre de confinement de gaz (14) jusqu'au patient lors de l'inhalation.

**13.** Dispositif (1) selon la revendication 12, dans lequel la force nécessaire pour courber ladite soupape à clapet résilient en direction de la personne est plus importante que la force nécessaire pour courber ladite soupape à clapet résilient dans la direction opposée à la personne.

**14.** Dispositif (1) selon la revendication 12 ou 13, dans lequel au moins une partie du clapet résilient est convexe par rapport à la personne.

**15.** Dispositif (1) selon la revendication 12, 13 ou 14, dans lequel au moins une partie du clapet résilient est concave par rapport à la direction de la cartouche de gaz (98).

**16.** Dispositif (1) selon l'une quelconque des revendications 12 à 15, dans lequel ladite soupape à clapet résilient permet à l'air de circuler sensiblement librement lors de l'expiration, tandis qu'elle ne permet qu'à un courant limité d'air de passer entre ladite circonférence de ladite soupape à clapet résilient et la surface interne de ladite paroi externe (38) lors de l'inhalation.

**17.** Dispositif (1) selon l'une quelconque des revendications précédentes, dans lequel ledit moyen coulissant (50, 250) entoure complètement ladite surface externe de ladite paroi (12) dudit récipient de gaz (10), d'une manière sensiblement étanche à l'air.

**18.** Dispositif (1) selon l'une quelconque des revendications précédentes, ledit dispositif (1) comprenant une paroi externe (38) agencée sous la forme d'un tube avec un diamètre sensiblement égal au diamètre de la cartouche de gaz (98), ladite paroi externe (38) entourant ladite entrée (16) et ladite sortie (18) dudit récipient de gaz (10), et ledit dispositif (1) comprenant un moyen d'espacement (36) pour relier ledit récipient de gaz (10) à ladite paroi externe (38) ; dans lequel ladite paroi externe (38) est reliée à ladite embouchure (22).

**19.** Dispositif (1) selon la revendication 18, dans lequel ladite paroi externe (38) entoure en outre au moins une partie de la cartouche de gaz (98).

**20.** Dispositif (1) selon la revendication 18 ou 19, dans lequel ladite paroi externe (38) est munie d'au moins une ouverture (40) située entre ladite entrée (16) et ladite sortie (18) dudit récipient de gaz (10), pour permettre à l'air de pouvoir sortir du dispositif (1) lors de l'expiration.

**21.** Dispositif (1) selon l'une quelconque des revendications précédentes, ledit dispositif (1) comprenant une enveloppe externe (42), ladite enveloppe externe (42) recouvrant au moins une partie de la partie inférieure de la cartouche de gaz (98), dans lequel ladite enveloppe (42) est adaptée pour s'ajuster de manière étroite sur la cartouche de gaz (98) et permettre le mouvement de ladite enveloppe externe (42) par rapport audit récipient de gaz (10), de sorte que le récipient de gaz (10) puisse être activé par le mouvement de ladite enveloppe (42).

**22.** Dispositif (1) selon la revendication 21, dans lequel ladite enveloppe externe (42) est munie d'une attache (44) permettant la fixation du dispositif (1) aux vêtements.

**23.** Dispositif (1) selon la revendication 21 ou 22, dans lequel la cartouche de gaz (98) est activée par la rotation de ladite enveloppe externe (42) par rapport audit récipient de gaz (10).

**24.** Dispositif (1) selon la revendication 23, dans lequel l'enveloppe externe (42) comprend une ouverture allongée (46), ladite ouverture allongée (46) ayant au moins une partie inclinée par rapport à la direction longitudinale de la cartouche de gaz (98), et ledit dispositif (1) comprend en outre une goupille d'activation (48) reliée audit récipient de gaz (10), ladite goupille (48) s'engageant dans ladite ouverture allongée (46) de sorte que la rotation de ladite enveloppe externe (42) par rapport audit récipient de gaz (10) active ladite cartouche de gaz (98).

25. Dispositif (1) selon l'une quelconque des revendications précédentes, dans lequel la surface externe dudit dispositif (1) est agencée sous la forme d'un cylindre.

26. Dispositif (1) selon l'une quelconque des revendications précédentes, dans lequel la chambre de confinement de gaz (14) est adaptée pour contenir du gaz à une pression supérieure à une pression choisie dans le groupe constitué de 1,05 ; 1,1 ; 1,2 ; 1,3 ; 1,5 ; 1,6 ; 1,7 ; 1,8 ; 1,9 ; 2 ; 2,5 et 3 atm.

27. Dispositif (1) selon l'une quelconque des revendications précédentes, dans lequel la chambre de confinement de gaz (14) est adaptée pour contenir un gaz à une pression inférieure à une pression choisie dans le groupe constitué de 4 ; 3 ; 2,5 ; 2 ; 1,5 ; 1,4 ; 1,3 ; 1,2 et 1,1 atm.

28. Dispositif (1) selon l'une quelconque des revendications précédentes, dans lequel la chambre de confinement de gaz (14) a des dimensions qui réduisent la vitesse du gaz sortant de la chambre (14) à une fraction de la vitesse du gaz entrant dans la chambre (14).

29. Dispositif (1) selon l'une quelconque des revendications précédentes, dans lequel la chambre de confinement de gaz (14) a des dimensions qui réduisent la vitesse du gaz sortant de la chambre (14) à une fraction de la vitesse de gaz entrant dans la chambre (14), ladite fraction étant inférieure à un nombre choisi dans le groupe constitué de 0,9 ; 0,8 ; 0,7 ; 0,6 et 0,55.

30. Dispositif (1) selon l'une quelconque des revendications précédentes, dans lequel la chambre de confinement de gaz (14) a des dimensions qui réduisent la vitesse du gaz sortant de la chambre (14) à une fraction de la vitesse du gaz entrant dans la chambre (14), ladite fraction étant supérieure à un nombre choisi parmi le groupe constitué de 0,1 ; 0,2 ; 0,3 ; 0,4 ; et 0,45.

31. Dispositif (1) selon l'une quelconque des revendications précédentes, dans lequel la chambre de confinement de gaz (14) a des dimensions qui réduisent la vitesse du gaz sortant de la chambre (14) à une fraction de la vitesse du gaz entrant dans la chambre (14), ladite fraction étant choisie dans le groupe constitué des intervalles 0,9 à 0,1 ; 0,8 à 0,2 ; 0,7 à 0,3 ; 0,6 à 0,et 0,55 à 0,45.

32. Dispositif (1) selon l'une quelconque des revendications précédentes, dans lequel la chambre de confinement de gaz (14) a un volume avec des dimensions du même ordre que les dimensions de la chambre d'aération à l'intérieur de la cartouche de gaz (98).

33. Dispositif (1) selon l'une quelconque des revendications précédentes, dans lequel la chambre de confinement de gaz (14) a un volume supérieur à un volume choisi dans le groupe constitué de 0,1 $cm^3$; 0,2 $cm^3$ ; 0,3 $cm^3$; 0,4 $cm^3$; 0, 5 $cm^3$; 0,6 $cm^3$ ; 0,7 $cm^3$; 0,8 $cm^3$; 0,9 $cm^3$ et 1 $cm^3$.

34. Dispositif (1) selon l'une quelconque des revendications précédentes, dans lequel la chambre de confinement de gaz (14) a un volume inférieur à un volume choisi dans le groupe constitué de 100 $cm^3$ ; 50 $cm^3$ ; 25 $cm^3$ ; 10 $cm^3$ ; 5 $cm^3$ ; 3 $cm^3$ ; 2 $cm^3$ ; 1, 7 $cm^3$ et 1,5 $cm^3$.

35. Dispositif (1) selon l'une quelconque des revendications précédentes, dans lequel la chambre de confinement de gaz (14) a un volume choisi dans le groupe constitué des intervalles 0,1 à 100 $cm^3$ ; 0,2 à 50 $cm^3$ ; 0,3 à 25 $cm^3$ ; 0,4 à 10 $cm^3$ ; 0,5 à 5 $cm^3$ ; 0,6 à 3 $cm^3$ ; 0 , 7 à 2 $cm^3$ ; 0,9 à 1, 7 $cm^3$ et 1 à 1,5 $cm^3$ .

36. Dispositif (1) selon l'une quelconque des revendications précédentes, muni de moyens d'enveloppe amovibles pour recouvrir ladite soupape de sortie (20), lorsque ledit dispositif (1) n'est pas utilisé pour l'inhalation.

Fig. 1

EP 1 453 563 B1

Fig. 3

Fig. 2

262   296

260

226   264

216

Fig. 4

238   212   232

230   256

250

246   248

220   234   224

Fig. 6   Fig. 5

**EP 1 453 563 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0064520 A
- EP 0311770 B1
- US 6293279 B
- US 6039042 A
- EP 0938908 A
- US 5042467 A
- US 4534343 A